# EUROPEAN PATENT APPLICATION

(11) **EP 4 056 680 A1**
(43) Date of publication of application: **14.09.2022**
(21) Application number: 20886117.9
(22) Date of filing: 05.11.2020
(51) Int. Cl.: C12N 5/073, C12N 5/0735, C12N 5/074

(54) **COLD STORAGE SOLUTION FOR STEM CELLS**

(30) Priority: 08.11.2019 JP 2019203124
(71) Applicant: Bioverde Inc., Kyoto-shi, Kyoto 601-8023 (JP)
(72) Inventor: AIZAWA, Akira, Kyoto-shi, Kyoto 6018023 (JP); HYON, Suong-Hyu, Kyoto-shi, Kyoto 6018023 (JP); HYON, Woogi, Kyoto-shi, Kyoto 6018023 (JP)
(74) Representative: Global IP Europe Patentanwaltskanzlei
(86) International application number: PCT/JP2020/041313
(87) International publication number: WO 2021/090869

(57) **Abstract**

A non-freezing refrigerated storage liquid for stem cells such as iPS cells, according to an embodiment, comprises: potassium ion species in a range of 30 to 80 mmoL/L; sodium ion species in a range of 30 to 80 mmoL/L, wherein ion-based molar ratio of sodium ion species to potassium ion species (ratio of Na⁺ to K⁺) is in a range of 0.5 to 1.3; trolox or its analog at a concentration of 1 to 8 mM; adenine or a salt or derivative thereof at a concentration of 0.1 to 4.2 mM; all of or all but one, two or three of essential amino acids, total content of which is 50 to 200 mg; and non-essential amino acids, total content of which is 50 to 200 mg/L.

## Description

The present invention relates to a storage liquid for preserving cells, tissues, organoids, organs, etc. in a non-frozen state. In particular, it relates to a refrigerated storage liquid for storage at a temperature lower than room temperature, for example, 2 to 15 °C, 2 to 8 °C. or 2 to 6 °C. Further, in particular, for storing, by suspending or immersing, pluripotent cells such as human iPS cells and other stem cells, derivatives such as organoids obtained from them, early embryos, etc.

Convenient, efficient and stable storage and supply of stem cells such as iPS and ES cells is an important technology that is indispensable in the fields of basic research and clinical application research or drug discovery research, in regenerative medicine. Cryopreservation of human iPS cells and ES cells has many problems such as: low survival rate after storage when the slow-freezing cryopreservation method is used; cytotoxicity of DMSO, which is a cryoprotective agent used for cryopreservation; and differentiation inducing capability. Non-freezing low-temperature storage of cells is a very attractive technique as a short-term (for example, 1 day to 2 weeks) to medium-term (for example, half a month to 3 months) preservation method, and in same time would be very advantageous in preservation of differentiation-induced cells and the preservation of organoids when the industrialization of regenerative medicine technology and its application to drug discovery are considered. However, it is difficult to cryopreserve pluripotent stem cells including human iPS cells using UW solution (UW_sln; BELZER UW^{®} COLD STORAGE LIQUID), which is widely used as a cryopreservation liquid for organs. Therefore, it is essential to develop a new refrigerated storage technology.

UW solution is widely used for refrigerated storage of organs used for transplantation such as liver and kidney, and it is practically a de facto standard or gold standard for such. The UW solution was developed in the 1980s and has a rather simple composition having an intracellular-type salt composition of low Na ion content and high K ion content, and containing lactobionat, raffinose, glutathione, hydroxyethyl starch.
PCT/JP2009/002941 (JP5726525B)
JP6220108B (JP2012-217342A)
WO2016/076317A
   - COMPARE BELZER UW^{™} COLD STORAGE LIQUID TO VIASPAN https://bridgetolife.com/compare-belzer-uw-cold-storage-solution-to-viaspan/
   - A novel efficient feeder-free culture system for the derivation of human induced pluripotent stem cells, Scientific Reports 4 : 3594 doi: 10.1038/srep03594
   - Cryopreservation and Hypothermal Storage of Hematopoietic Stem Cells https://etd.ohiolink.edu/! etd.send_file?accession=ucin1439307244&disposition=inline

However, when to store cells or tissues in a non-freezing refrigerated state, UW solution cannot sufficiently suppress low-temperature storage stress. To solve this problem, the inventors of the present invention have hypothesized that it was important to adopt an intermediate between: the intracellular type compositions represented by the UW solution; and extracellular type compositions having high Na ion content and low K ion content that are common in generally used culture media. In addition, we have conceived and idea that the addition of macromolecules such as proteins is important on contrary to organ preservation. Thus, the inventors has investigated storage liquids that have compositions at an intermediate between the UW solution and the cell culture media.

There are few reports on the low-temperature storage medium for cells. There are only a few reports of storage by using recently developed HTS FRS (Hypothermosol^{®} FRS; BioLife Solutions). It has been reported that this HTS FRS can store human skin fibroblasts and smooth muscle cells at the low temperature for 3 to 5 days, but there is no report on the non-freezing refrigerated storage of pluripotent cells such as human iPS cells.

While trying a wide variety of combinations, the inventors of the present invention have adopted mixing, at a ratio of about 1/1 to 1/2: MEM-alpha (Minimum Essential Medium Eagle (MEM) Alpha Modification) or StemFit^{®} AK02N (Ajinomoto Co., Inc.); with the above UW solution or a "ES cells culture liquid", which is a modification based on DMEM: F12 medium as described later and hereinafter occasionally referred to as "ESC medium". The MEM-alpha is a modification based on MEM medium and contains non-essential amino acids, sodium pyruvate, lipoic acid, biotin and vitamin B 12.

The MEM (Eagle's minimal essential medium) medium contains the following components (1) to (4).
(1) Amino acids (L-arginine, L-cystine, L-glutamine, L-histidine, L-isoleucine, L-leucine, L-lysine, L-phenylalanine, L-threonine, L-tryptophan, L-tyrosine, L -Valin),
(2) Salts (calcium chloride, potassium chloride, magnesium sulfate, sodium chloride, sodium dihydrogen phosphate),
(3) D-glucose and
(4) Vitamins (folic acid, nicotinamide, riboflavin, B12, choline, inositol, pantothenic acid, pyridoxal phosphate, thiamine).

On the other hand, the UW solution is a Ca²⁺-free solution having an intracellular fluid composition, and contains: lactobionic acid and hydroxy-ethyl starch (HES) for adjusting osmotic pressure and suppressing cell expansion; adenosine as anti-inflammatory and vascular relaxation agent and as a precursor of ATP; glutathione and alloprinol for antioxidants; and a phosphate buffer as a buffer component.

StemFit^{®} AK02N is a feederless culture medium for human iPS cells, consisting of 400 mL of solution "A", 100 mL of solution "B", and 2 mL of solution "C", and is used by mixing immediately before use. At a time of mixing the solutions "A" to "C", the above-mentioned UW solution or "ES cells culture liquid" was also admixed with them. The solution "C" is a physiological buffer solution in which a basic fibroblast growth factor (bFGF) is dissolved.

When compared with MEM-alpha, StemFit^{®} AK02N can be said to have the same salt composition, the essential amino acid composition is almost the same, and the non-essential amino acid composition is largely the same. As suggested by the experimental results described later, MEM-alpha and StemFit^{®} AK02N could be used in almost the same manner in the refrigerating storage liquid of the present invention. Common features among these two media is considered to be important for the non-freezing refrigerated storage liquid for stem cells according to present invention, as suggested by experimental data presented later. The common features here are compositions of salts and essential amino acids and major part of composition of the non-essential amino acids. On the other hand, the main difference between MEM-alpha and StemFit^{®} AK02N is that both proteins and growth factors are contained only in StemFit^{®} AK02N. It is presumed that these components are not essential and do not contribute much to the refrigerating storage liquid of the present invention. While StemFit^{®} AK02N is a culture medium for iPS cells, MEM-alpha cannot culture iPS cells even if bFGF, which is a typical growth factor, is added. Similarly, the ES cell culture medium ("ESC medium") cannot culture iPS cells without feeder cells.

The DMEM: F12 medium, which is the base of the ES cell culture medium ("ESC medium"), is a mixture of MEM (Eagle's minimal essential medium) medium and Ham's F12 medium in a volume ratio of 1/1. The Ham's F12 medium is a serum-free basal media containing putrescine, hypoxanthine and thymidine.

The non-freezing refrigerated storage liquid according to a preferred embodiment of the present invention has features A1 to A6 or A1 to A9 at below.
A1. Content of potassium ion species is 20 to 90 mmoL/L or 30 to 80 mmoL/L, and the content of sodium ion species is 20 to 90 mmoL/L or 30 to 80 mmoL/L.
A2. Ion-based molar ratio (Na⁺ / K⁺ ratio) of sodium ion species to potassium ion species is 0.5 to 1.5, 0.5 to 1.3, or 0.6 to 1.2.
A3. Trolox or its analog is contained at concentrations of 0.5-10 mM, 1-8 mM or 2-7 mM.
A4. Adenine or a salt or derivative thereof is contained at a concentration of 0.1 to 4.2 mM, 0.1 to 6 mM, 1 to 6 mM, 2 to 5 mM or 3 to 5 mM.
A5. All or all but one, two or three essential amino acids are contained, and the total content of essential amino acids is preferably 50 to 250 mg/L, particularly 50 to 200 mg/L or 80 to 150 mg/L.
A6. Total content of non-essential amino acids is preferably 100 to 500 mg/L, especially 50 to 200 mg/L or 80-150 mg/L.
A7. Content of magnesium ion species (particularly in magnesium sulfate) is 2 to 8 mmoL/L, 2 to 5 mmoL/L or 2-4 mmoL/L, and the calcium content is 0.2 to 1.5 mmoL/L, 0.2 to 1 mmoL/L or 0.3 to 0.8 mmoL/L.
A8. Glucose or other low-molecular-weight polysaccharides (particularly, disaccharides such as maltose, and monosaccharides such as fructose) is contained at a concentration of 0.5 to 10 mmoL/L.
A9. Vitamins at below are contained whereas one, two or three among the below can be omitted.

Folic acid, nicotinamide, riboflavin, B12, choline, inositol, pantothenic acid, pyridoxal phosphate, and thiamine.

The non-freezing refrigerated storage liquid according to a preferred embodiment of the present invention is a physiological solution, pH of which is set in a range of 7 to 7.5, and comprises:
(i) lactobionic acid or a salt thereof (lactobionate) in terms of lactone, in a range of 30 to 100 mmoL/L (or 10 to 40 g/L),
(ii) raffinose hydrate in a range of 10 to 30 mmoL/L (or 5 to 20 g/L),
(iii) alloprinol in a range of 0.3 to 1 mmoL/L (or 0.05 to 0.1 g/L),
(iv) glutathione (total glutathione) in a range of 1 to 3 mmoL/L,
(v) adenosine in a range of 2 to 10 mmoL/L (or 0.3 to 0.9 g/L),
(vi) lipoic acid in a range of 0.05-1 µmoL/L (or 0.03 to 0.2 mg/L),
(vii) sodium pyruvate in a range of 0.1 to 1 mmoL/L (or 10 to 100 mg/L),
(viii) glucose in a range of 0.5 to 10 mmoL/L (or 100 to 1000 mg/L),
(ix) ascorbic acid in a range of 0.03 to 0.3 mmoL/L, and vitamin E or its water-soluble analog/derivative in a range of 1 to 10 mM,
(x) adenine or its salt or derivative, in a range of 0.1 to 4.2 mM,
(xi) other vitamins or water-soluble analog/derivative thereof,
(xii) essential amino acids in a range of 50 to 200 mg/L in total,
(xiii) non-essential amino acids in a range of 100 to 500 mg/L in total,
(xiv) potassium ion species in a range of 30 to 80 mmoL/L, and
(xv) sodium ion species in a range of 20 to 90 mmoL/L.

This non-freezing refrigerated storage liquid preferably further comprises:
(xv) hydroxyethyl starch in a range of 20 to 50 g/L; and/or
(xvi) ribonucleoside in a range of 4 to 40 mg/L, and/or
(xvii) deoxyribonucleoside in a range of 4-40 mg/L; and/or
(xviii) potassium dihydrogen phosphate and sodium dihydrogen phosphate, in a range of 10 to 25 mmoL/L.

In occasions of non-freezing refrigerated storage of pluripotent cells such as human iPS cells, it is able to maintain a high viability maintenance effect and proliferative ability after recultivation.
FIG. 1A is a graph showing a change in relative viability depending on the number of storage days when the survival rate at the start of storage is taken as 1.0, and when stored at 4 °C in a single cell state. In the experiments shown in FIGS. 1 to 7F, a non-freezing refrigerated storage medium ("HTM1"), which was obtained by mixing the UW solution and the clinical-research use medium of StemFit^{®} AK02N, at a ratio of 2/1.
FIG. 1B is a bar graph showing 50% survival-rate maintenance days (ST50) as obtained from the graph of FIG. 1A.
FIG. 2A is a graph showing a change in survival rate depending on the number of storage days when stored at 4 °C in a single cell state, as in FIG. 1A while, as in FIG. 1A, the survival rate at the start of storage is taken as 1.0.
FIG. 2B is a bar graph showing 80% survival-rate maintenance days (ST80) as obtained from the graph of FIG. 2A.
FIG. 2C is a bar graph showing colony forming ability subsequent to culturing for 5 days, after non-freezing refrigerated storage for 6 days.
FIG. 3A is a bar graph showing the rate of cell death due to apoptosis that occurs immediately after the non-freezing refrigerated storage for 3 days.
FIG. 3B is a bar graph showing the rate of cell death due to necrosis that occurs immediately after refrigerated storage for 3 days as in FIG. 3A.
FIG. 4A is a bar graph showing changes in intracellular reactive oxygen species (ROS) concentration that occur immediately after the non-freezing refrigerated storage for 3 days in the same manner as in FIGS. 3A to 3B.
FIG. 4B is a bar graph showing changes in the activity of intracellular caspase 3/7, which is an index of apoptosis, immediately after the non-freezing refrigerated storage for 3 days in the same manner as in FIGS. 3A to 3B and FIG. 4A.
FIG. 5A summarizes the chemical formulas of the radical scavengers used in the experiment.
FIG. 5B is a bar graph showing the 80% survival-rate maintenance days (ST80) as in FIG. 2B when various radical scavengers shown in FIG. 5A were added.
FIG. 6A is a graph showing a change in survival rate depending on the number of storage days, similar to FIG. 2A when the addition concentration of trolox, which is a radical scavenger, is changed.
FIG. 6B is a bar graph showing 80% survival-rate maintenance days (ST80) as obtained from the graph of FIG. 6A.
FIG. 6C is a bar graph showing colony forming ability subsequent to culturing for 5 days, after non-freezing refrigerated storage for 6 days, in each of media having varied concentration of trolox.
FIG. 6D is a bar graph showing effects of adding tohophenol or tocopherol acetate on the relarive viability obtained subsequent to recultivation for 1 days after refrigerated storage for 6 days.
FIG. 7A is a phase difference image showing colonies obtained subsequent to recultivation for 4 days after refrigerated storage for 3 days.
FIG. 7B is a binarized image of FIG. 7A.
FIG. 7C is a phase difference image showing colonies obtained subsequent to recultivation for 4 days after refrigerating for 6 days.
FIG. 7D is a binary version of the image of FIG. 7B.
FIG. 7E is a bar graph showing the area ratio of colonies in a medium recultivated for 4 days after non-freezing refrigerated storage for 3 days and 6 days.
FIG. 7F is a bar graph showing growth rates in numbers of colonies when reculivated for 4 days after non-freezing refrigerated storage for 3 days and 6 days, as expressed as ratios to that of the non-preservation control (as taken as 1), which was cultured for 4 days without refrigerated storage.
FIG. 8 is a bar graph showing relative growth rates of FIG. 7F along with the rate when trolox or the like is omitted, and the rates when added with Trolox 5 mM and adenine as varied from 1.4 to 8.2 mM.
FIG. 9 is a bar graph in a manner of FIGS. 7F and 8, showing growth rates in numbers of colonies when reculivated for 4 days after non-freezing refrigerated storage for 7days, by adopting a non-freezing refrigerated storage medium "HTM-alpha" (UW solution + MEM-alpha, 2:1) in which UW solution and MEM-alpha are mixed at a ratio of 2/1. Here, 5 mM Trolox and 5-fold concentration (x5; 10 mM) of GlutaMAX^{®} (registered trademark, GIBCO) were added to "HTM-alpha" and "HTM1". A result of using HTS FRS, which is a commercially available refrigerated storage medium, is also shown.
FIG. 10 shows rates at which mouse blastocyst stage embryos (3.5 days after in vitro fertilization) escaped from the zona pellucida during a 2-day culture period, after being stored as non-freezing refrigerated for 3 days in storage media same as in FIG. 9.
FIG.11 is a bar graph in a manner of FIG. 10, showing effects of varied concentrations of adenine hydrochloride under conditions same as in the experiment of FIG. 10.
FIG.12A is a bar graph showing protective effects of polyvinyl alcohol (PVA) in place of hydroxyl ethyl starch in an experiment same manner with that of FIG.6A.
FIG.12B is a bar graph showing effects of further adding mannitol in addition to polyvinyl alcohol (PVA) in an experiment same manner with that of FIG.12A.

The non-freezing refrigerated storage liquid according to a preferred embodiment of the present invention is a physiological buffer solution, pH of which is set to 7 to 7.5, comprises (i) to (vi) at below.
(i) Lactobionic acid or a salt thereof (lactobionate) in terms of lactone 20-100 mmoL/L, 30-100 mmoL/L, 30-80 mmoL/L or 30-60 mmoL/L, or 10-50 g/L, 10-40g/L, 15-35g/L or 20-30g/L.
(ii) Raffinose hydrate 10-40 mmoL/L, 10-30 mmoL/L or 10-20 mmoL/L, or 5-20 g/L or 8-15 g/L.
(iii) Allopurinol 0.3 to 2 mmoL/L, 0.3 to 1 mmoL/L or 0.4 to 0.8 mmoL/L, or 0.03 to 0.15 g/L, 0.05 to 0.1 g/L or 0.06 to 0.08 g/L.
(iv) Glutathione (total glutathione) 1-3 mmoL/L or 1.5-2.5 mmoL/L, or 0.3-1 g/L, 0.4-0.9 g/L or 0.4-0.8 g/L.
(v) Adenosine 2-10 mmoL/L, 2-8 mmoL/L or 2-5 mmoL/L, or 0.5-1.5 g/L or 0.5-1.3 g/L.
(vi) Lipoic acid 0.05 to 1 µmoL/L, 0.1 to 0.5 µmoL/L or 0.2 to 0.4 µmoL/L, or 0.03 to 0.2 mg/L or 0.05 to 0.1 mg/L.
(vii) Sodium pyruvate 0.1 to 1 mmoL/L, 0.1 to 0.7 mmoL/L or 0.2 to 0.5 mmoL/L, or 10 to 100 mg/L or 20 to 50 mg/L.
(viii) Glucose 0.5 to 10 mmoL/L, 1 to 5 mmoL/L or 1 to 3 mmoL/L, or 100 to 1000 mg/L or 200 to 500 mg/L.
(ix) antioxidant vitamins or their water-soluble analogs/derivatives, in the following.

Each in the following take a form of salt. In some cases, ix-1 may be omitted.
ix-1. Ascorbic acid 0.03 to 0.3 mmoL/L, 0.05 to 0.2 mmoL/L or 0.05 to 0.15 mmoL/L, or 5 to 20 mg/L or 10 to 15 mg/L.
ix-2. Vitamin E, trolox or other water-soluble vitamin E analog/derivatives 0.5-10 mM, 1-8 mM or 2-7 mM.
(x) Adenine or its salt or derivative 0.1-6 mM, 0.1-5 mM, 0.1-4.5 mM, 0.1-4.2 mM or 2-5 mM, and in some cases, 0.1-0.3 mM or 0.05-0.2 mM.
(xi) Other vitamins not mentioned above, or water-soluble analog derivatives thereof

Each in the following may take a form of salt. And, out of ten compounds in the following, one to five compounds, one to four compounds, or one to three compounds may be omitted.
xi-1. Biotin 0.03 to 0.25 µmoL/L, 0.05 to 0.2 µmoL/L or 0.1 to 0.15 µmoL/L, or 0.01 to 0.1 mg/L or 0.02 to 0.05 mg/L.
xi-2. Vitamin B12 0.03 to 3 µmoL/L, 0.05 to 2 µmoL/L or 0.1 to 0.5 µmoL/L, or 0.1 to 0.8 mg/L or 0.2 to 0.6 mg/L.
xi-3. Folic acid 0.2-2 µmoL/ L, 0.3-1 µmoL/L or 0.5-0.9 µmoL/L, or 0.1-1 mg/L or 0.2-0.5 mg/L.
xi-4. Niacinamide 0.5-8 µmoL/L, 1-6 µmoL/L or 1-4 µmoL/L, or 0.03-0.2 mg/L or 0.05-0.1 mg/L.
xi-5. Riboflavin 0.03 to 0.3 µmoL/L, 0.04 to 0.2 µmoL/L or 0.05 to 0.15 µmoL/L, or 0.03 to 0.2 mg/L or 0.05 to 0.1 mg/L.
xi-6. Choline 0.05 to 1 µmoL/L, 0.1 to 0.5 µmoL/L or 0.1 to 0.4 µmoL/L, or 0.1 to 1 mg/L or 0.2 to 0.5 mg/L.
xi-7. Inositol 1-10 µmoL/L, 1-8 µmoL/L or 2-6 µmoL/L, or 0.03-0.2 mg/L or 0.05-0.1 mg/L.
xi-8. Pantothenic acid 0.02 to 0.2 µmoL/L, 0.03 to 0.1 µmoL/L or 0.04 to 0.08 µmoL/L, or 0.1 to 1 mg/L or 0.2 to 0.5 mg/L.
xi-9. Pyridoxal 0.5 to 3 µmo L/L, 0.1 to 2 µmo L/L or 1 to 2 µmo L/L, or 0.1 to 1 mg/L or 0.2 to 0.5 mg/L.
xi-10. Thiamine 0.3 to 3 µmoL/L, 0.4 to 2 µmoL/L or 0.5 to 1.5 µmoL/L, or 0.1 to 1 mg/L or 0.2 to 0.5 mg/L.
(xii) Essential amino acids 50-200 mg/L or 80-150 mg/L in total.

Amount of each component may be in a range of A/3 to 3A or of A/2 to 2A, where A is the value obtained by dividing by 3, the content shown in Table 2 or the value in "HTM-alpha" in Examples mentioned later. That is, amount of each component may be set in a range of 1/3 to 3 times, or in a range of 1/2 to 2 times, of the value in "HTM-alpha". The same applies to the dictated non-essential amino acids and the vitamins mentioned above.
xii-1. Isoleucine
xii-2. Leucine
xii-3. Ricin
xii-4. Methionine
xii-5. Phenylalanine
xii-6. Threonine
xii-7. Tryptophan
xii-8. Valine
xii-9. Histidine
(xiii) Non-essential amino acids in the following, 100-500 mg/L or 150-400 mg/L in toral.

Each in the following may take a form of salt. And, out of ten compounds in the following, one to five compounds, one to four compounds, or one to three compounds may be omitted.
xiii-1. Glycine
xiii-2. Alanine
xiii-3. Arginine
xiii-4. Asparagine
xiii-5. Aspartic acid
xiii-6. Cysteine
xiii-7. Cystine
xiii-8. Glutamic acid
xiii-9. Glutamine
xiii-10. Proline
(xiv) Potassium ion species 20-90 mmoL/L, 30-80 mmoL/L, 40-80 mmoL/L or 50-70 mmoL/L. Namely, 20 mmoL/L or more, 30 mmoL/L or more, 40 mmoL/L or more, or 50 mmoL/L or more, and less than 90 mmoL/L, less than 80 mmoL/L, or less than 70 mmoL/L.
(xv) Sodium ion species 20-90 mmoL/L, 30-80 mmoL/L, 40-80 mmoL/L or 50-70 mmoL/L. Namely, 20 mmoL/L or more, 30 mmoL/L or more, 40 mmoL/L or more, or 50 mmoL/L or more, and less than 90 mmoL/L, less than 80 mmoL/L, or less than 70 mmoL/L.

This non-freezing refrigerated storage liquid preferably contains at least one of the following (xvi) to (xviii). Particularly preferably, the following (xvi) to (xvii) and the following (xviii) are included.
(xvi) Hydroxyethyl starch 10-80 g/L, 20-50 g/L or 20-40 g/L.
(xvii) Polyvinyl alcohol 3-100g/L, 5-80g/L, 5-50g/L, 5-30g/L, 5-25g/L, or 5-20g/L.
(xviii) Mannitol 20-100 mmoL/L, 30-90 mmoL/L or 20-90 mmoL/L.

This non-freezing refrigerated storage liquid preferably contains at least one of the following (xix) and (xx).
(xix) Ribonucleoside 2-4 types Total 4-40 mg/L, 5-30 mg/L or 10-15 mg/L.
(xx) Deoxyribonucleosides 2-4 types Total 4-40 mg/L, 5-30 mg/L or 10-15 mg/L.

This non-freezing refrigerated storage liquid preferably contains the following (xxi) to (xxiii).
(xxi) Potassium dihydrogen phosphate or sodium dihydrogen phosphate 10-30 mmoL/L, 10-25 mmoL/L, or 15-25 mmoL/L.
(xxii) Magnesium ion species (especially as magnesium sulfate) 2 to 8 mmoL/L, 2 to 5 mmoL/L or 2 to 4 mmoL/L.
(xxiii) Calcium ion species (especially as magnesium chloride) 0.2 to 1.5 mmoL/L, 0.2 to 1 mmoL/L or 0.3 to 0.8 mmoL/L.

Further, according to a preferred embodiment of the present invention, in term of molar ratio (ratio of Na/K ions), amount of the sodium ion species is, for example, 0.7 to 1.3 times, 0.8 to 1.2 times, or 0.9 to 1.1 times that of the potassium ion species. According to another preferred embodiment, amount of the sodium ion species may be, for example, 0.5 to 1.5 times, 0.5 to 1.3 times, or 0.6 to 1.2 times of the potassium ion species, in the term of molar ratio.

As described above, the non-freezing refrigerated storage liquid according to the preferred embodiment of the present invention contains trolox or other water-soluble vitamin analogs in an amount of 0.1 mM or more, 0.3 mM or more, 0.5 mM or more, 1 mM or more, 2 mM or more, 3 mM, 4 mM or more, or 5 mM or more; and 10 mM or less, 8 mM or less, or 6 mM or less.

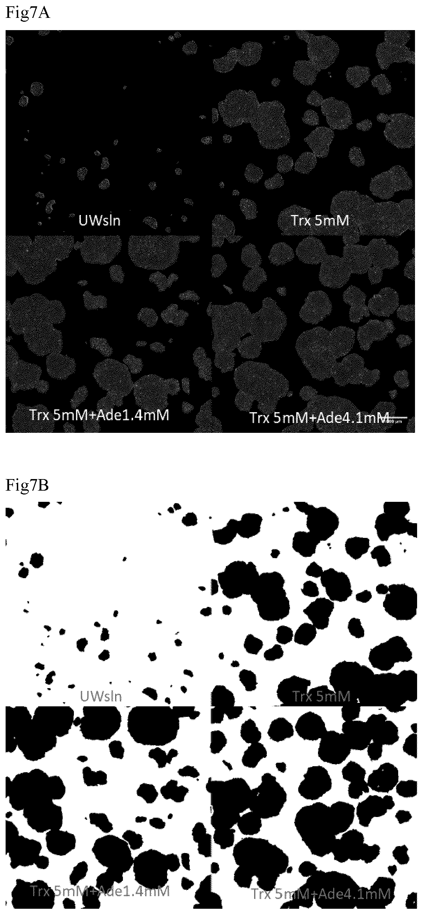

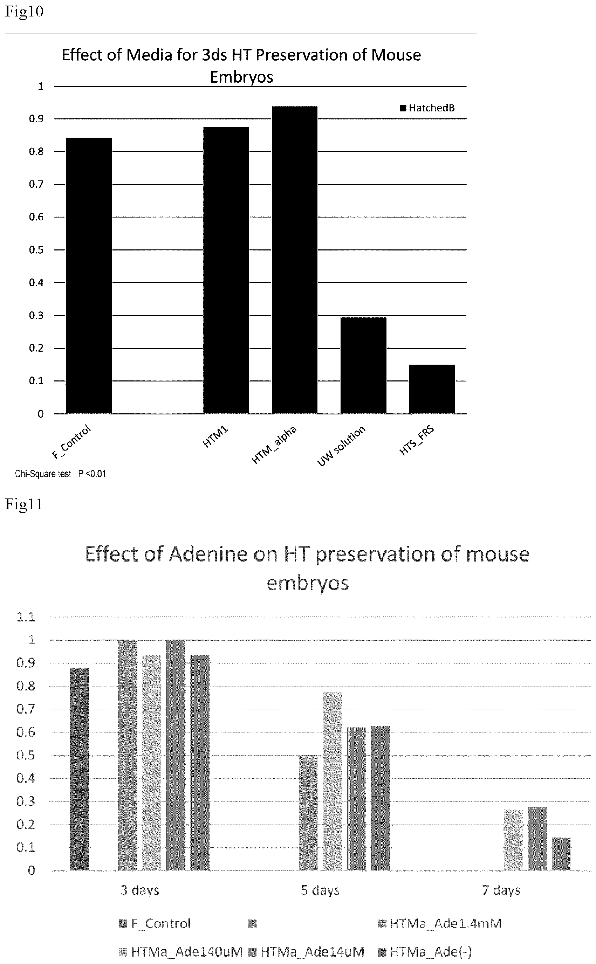

As described above, the non-freezing refrigerated storage liquid according to the preferred embodiment of the present invention contains, in particular, adenine or a salt thereof at 0.1 mM or more, 0.3 mM or more, 0.5 mM or more, 1 mM or more, 2 mM or more, 3 mM or more; and 8 mM or less, 6 mM or less, 5 mM or less or 4 mM or less; and, for example, 0.1 to 4.2 mM or 1 to 4.2 mM. In particular, it is considered that the addition of adenine has a remarkable effect in preserving embryos and organoids in which cells are adhered to each other to form a three-dimensional mass. In addition, in preserving embryos and organoids, effects were observed even at relatively low concentrations, for example, 0.01 to 0.2 mM (10 to 200 µM) or 0.05 to 0.3 mM (5 to 300 µM) (FIG. 11). On the other hand, when culturing stem cells such as iPS cells without forming embryos or organoids, it is considers as particularly effective to add adenine at a concentration of, for example, 0.5 to 6 mM, particularly 1 to 5 mM or 2 to 4 mM (FIGS. 7A-8).

The non-freezing refrigerated storage liquid according to the preferred embodiment of the present invention further comprises L-alanyl-L-glutamine, or other dipeptide substitute, or a salt thereof, at a concentration of 1 to 6 mM, 2 to 5 mM, or 3 to 5 mM.

The refrigerated storage liquid according to a preferred embodiment of the present invention is obtainable by mixing a UW solution having a composition shown in Table 1 with the MEM alpha medium having a composition shown in Table 2, so that amount of the UW solution is 1 to 3 times, preferably 1.5 to 2.5 times, more preferably 1.8 to 2.2 times of amount of the MEM alpha medium, in a volume ratio. The compositions of Tables 1 and 2 and the content of each component obtained from the above mixing ratio may be increased or decreased within a range of ± 50%, ± 40%, ± 30%, or ± 20% if necessary or appropriate. In addition, out of the components listed in Table 2, some components are omittable. Particularly omittable are: some of the non-essential amino acids (for example, 1 to 5 amino acids); some of the nucleosides (for example, 1 to 5 nucleosides); some vitamins (for example, 1 to 5 vitamins); some inorganic salts (for example, 1 to 3 salts), and phenol red.

A non-freezing refrigerated storage method according to a preferred embodiment of the present invention comprises: dispersing pluripotent cells in any of the above refrigerated storage liquids so that the cells are dispersed in a single cell state in a range of 1×10³ cells/mL to 1×10 cells/mL, or 1×10⁴ cells/mL to 1×10⁸ cells/mL; and storing in non-freezing refrigerated state for 1 to 10 days or 1 to 7 days, for example.

A non-freezing refrigerated storage method according to a preferred embodiment of the present invention comprises: seeding pluripotent cells into a culture container containing any of the above refrigerated storage liquids in a range of 1×10³ cells/cm² to 1×10⁹ cells/cm² or 1×10⁴ cells/cm² to 1×10⁸ cells/cm²; culturing for 2 to 6 days in such state; and then storing in non-freezing refrigerated state for 1 to 10 days or 1 to 7 days, for example.

According to a preferred embodiment of the present invention, the pluripotent cells or stem cells to be stored in a non-freezing refrigerated state are pluripotent cells such as ES cells and induced pluripotent stem cells such as iPS cells, or hematopoietic stem cells, nerve stem cells, and liver stem cells, skin stem cells, reproductive stem cells, etc. In addition, the pluripotent cells to be stored in a non-freezing refrigerated state may be of human origin or originated from mammals such as primates, mice and guinea pigs.

Further, according to another embodiment, target of non-freezing refrigerated storage may be human or animal embryos, particularly embryos of domestic animals such as cows, horses, pigs and goats.

### Examples

### 1.1. Preparation of cells for non-freezing refrigerated storage experiments

A healthy human-derived human iPS cell line (253G1) obtained from the Center for iPS Cell Research and Application, Kyoto University was maintained and cultured under feeder-free conditions. For this culture, StemFit (registered trademark) AK02N (Ajinomoto), which is a commercially available medium for clinical research for human ES/iPS cells was used. When the cells reached 70-80% confluence on the 7th day after the start of the culture, the cells were enzymatically stripped off from a container by using 0.5X TrypLE^{®} Select, and then dispersed and washed. Subsequently, the cells were suspended in a new StemFit^{®} AK02N medium containing 10 µM Y27362 (Fujifilm Wako Pure Chemical Industries, Ltd.) as a ROCK inhibitor, and added with iMatrix-511 (MATRIXOME, Inc.) as a further cell culture substrate at 0.1 µg/cm². Then, the cells were seeded at a concentration of 1.3E3/cm² (1.3×10³/cm²). The day after the seeding, the medium was replaced with StemFit^{®} AK02N medium containing no Y27362, then the medium was replaced every 3 days and subcultured or tested on day 7.

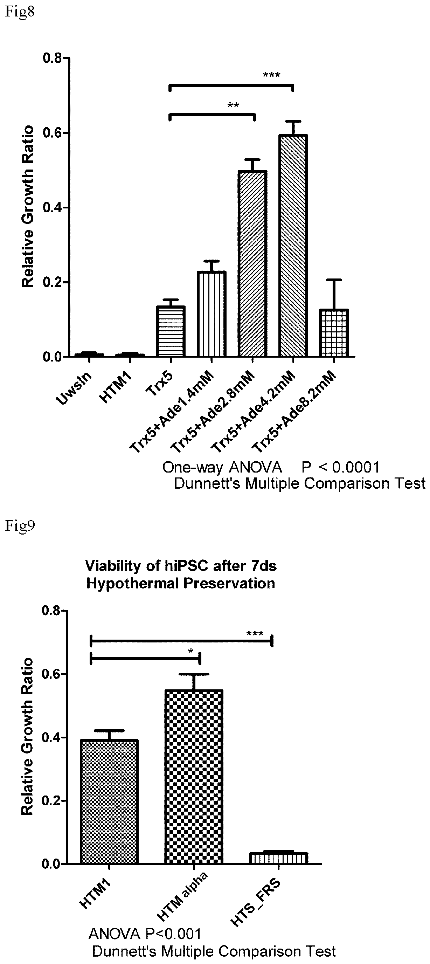

For the experiment using the following HTM-alpha as the non-freezing refrigerated storage medium (Fig. 9), the MEM-alpha medium used for the above HTM-alpha was used in place of StemFit (registered trademark) AK02N. Except for this, the preparation of the cells for the storage experiments was made as in the above.

On the other hand, in order to try non-freezing refrigerated storage of mouse embryos (Figs. 10 to 11), in vitro fertilization was performed by ova and sperms from female and male mice (both ICR), and then embryos were cultured for 3.5 days (84 hours). Here, HTF (Fuji Film Wako Pure Chemical Industries, Ltd.) was used as the medium for insemination, and KSOM (Arc Resource) was used for culturing.

In addition, in present application, unless otherwise specified, culturing was carried out in a 37 °C incubator (5% CO₂).

### 2. Non-freezing refrigerated storage liquid

The following 3 types of refrigerated storage liquids were prepared. For convenience, they will be referred to as HTM1, HTM2, and HTM-alpha, respectively.
- HTM1: UW solution + above-mentioned clinical research medium StemFit^{®} AK02N, mixing volume ratio: 2/1.
- HTM2: UW solution + the below-mentioned human ES cell culture solution ("ESC medium"), mixed volume ratio: 2/1.
- HTM-alpha: UW solution + MEM-alpha (12571--MEM alpha, nucleosides, Thermo Fisher Scientific), mixed volume ratio: 2/1. Before use, trolox was added to becomes 5 mM, and make it GlutaMAX (registered trademark, GIBCO) was added to become 5 times of standard concentration (x5; 10 mM).

Any of the following three types of refrigerated storage liquids was used as the refrigerated storage liquid of the comparative example.
- UW solution (BELZER UW (registered trademark) COLD STORAGE LIQUID)
- The above StemFit (registered trademark) AK02N
- HTS FRS (HypoThermosol^{®} FRS, BioLife Solutions)

Human ES cell culture medium ("ESC medium") is obtainable by adding components listed below into DMEM-F12 (Invitrogen 21331-020). At below, "%" and "mM" below indicate the weight ratio or molar concentration with respect to the finally obtained human ES cell culture medium.
- Non-essential amino acids (NON-ESSENTIAL AMINO ACID (×100), invitrogen 11140) 1%,
- 200 mM L-glutamine (L-GLUTAMINE (×100), invitrogen 25030) 1%,
- StemSure (registered trademark) Serum Replacement (SSR) (Fujifilm Wako Pure Chemical Industries, Ltd.) 20%,
- 2-MERCAPTOETHANOL (×1000; concentration at the time of addition) 0.1 mM,
- Penicillin and streptomycin (penicillin / streptomycin).

The composition of the UW solution (BELZER UW (registered trademark) COLD STORAGE LIQUID) is as shown in the table below according to Non-Patent Document 1 above.

**[Table 1] Composition of UW solution**

| Ingredient | G/L | MMOL/L |
|---|---|---|
| Hydroxyethyl starch | 50.0 | NA |
| Lactobionic acid (lactone equivalent) | 35.83 | 105 |
| Potassium dihydrogen phosphate | 3.4 | 25 |
| Magnesium Sulfate Hydrate | 1.23 | 5 |
| Raffinose pentahydrate | 17.83 | 30 |
| Adenosine | 1.34 | 5 |
| Allopurinol | 0.136 | 1 |
| Total glutathione | 0.922 | 3 |
| Potassium hydroxide | 5.61 | 100 |
| Sodium hydroxide / hydrochloric acid | (for adjusting pH to 7.4) | |
| Water for injection | appropriate amount | |

Specifically, the MEM alpha medium (12571--MEM alpha, nucleosides, Thermo Fisher Scientific) used in "HTM-alpha" has the following composition (https://www.thermofisher.com/jp). /en/home/technical-resources/media-formulation.94.html).

**[Table 2] Composition of MEM alpha medium**

| **Components** | **Molecular Weight** | **Concentration (mq/L)** | **mM** |
|---|---|---|---|
| **Amino Acids** | | | |
| **Glycine** | **75.0** | **50.0** | **0.6666667** |
| **L-Alanine** | **89.0** | **25.0** | **0.28089887** |
| **L-Arqinine hydrochloride** | **211.0** | **105.0** | **0.49763033** |
| **L-Asparaqine-H2O** | **150.0** | **50.0** | **0.33333334** |
| **L-Aspartic acid** | **133.0** | **30.0** | **0.22556391** |
| **L-Cysteine hydrochloride-H2O** | **176.0** | **100.0** | **0.5681818** |
| **L-Cystine 2HCl** | **313.0** | **31.0** | **0.09904154** |
| **L-Glutamic Acid** | **147.0** | **75.0** | **0.5102041** |
| **L-Glutamine** | **146.0** | **292.0** | **2.0** |
| **L-Histidine** | **155.0** | **31.0** | **0.2** |
| **L-Isoleucine** | **131.0** | **52.4** | **0.4** |
| **L-Leucine** | **131.0** | **52.0** | **0.39694658** |
| **L-Lysine** | **183.0** | **73.0** | **0.3989071** |
| **L-Methionine** | **149.0** | **15.0** | **0.10067114** |
| **L-Phenylalanine** | **165.0** | **32.0** | **0.19393939** |
| **L-Proline** | **115.0** | **40.0** | **0.3478261** |
| **L-Serine** | **105.0** | **25.0** | **0.23809524** |
| **L-Threonine** | **119.0** | **48.0** | **0.40336135** |
| **L-Tryptophan** | **204.0** | **10.0** | **0.04901961** |
| **L-Tyrosine disodium salt** | **225.0** | **52.0** | **0.23111111** |
| **L-Valine** | **117.0** | **46.0** | **0.3931624** |

| **Vitamins** | | | |
|---|---|---|---|
| **Ascorbic Acid** | **176.0** | **50.0** | **0.2840909** |
| **Biotin** | **244.0** | **0.1** | **4.0983607E-4** |
| **Choline chloride** | **140.0** | **1.0** | **0.007142857** |
| **D-Calcium pantothenate** | **477.0** | **1.0** | **0.002096436** |
| **Folic Acid** | **441.0** | **1.0** | **0.0022675737** |
| **Niacinamide** | **122.0** | **1.0** | **0.008196721** |
| **Pyridoxal hydrochloride** | **204.0** | **1.0** | **0.004901961** |
| **Riboflavin** | **376.0** | **0.1** | **2.6595744E-4** |
| **Thiamine hydrochloride** | **337.0** | **1.0** | **0.002967359** |
| **Vitamin B12** | **1355.0** | **1.36** | **0.0010036901** |
| **i-Inositol** | **180.0** | **2.0** | **0.011111111** |

| **Inorqanic Salts** | | | |
|---|---|---|---|
| **Calcium Chloride (CaCl₂) (anhyd.)** | **111.0** | **200.0** | **1.8018018** |
| **Maqnesium Sulfate (MgSO₄) (anhyd.)** | **120.0** | **97.67** | **0.8139166** |
| **Potassium Chloride (KCl)** | **75.0** | **400.0** | **5.3333335** |
| **Sodium Bicarbonate (NaHCO₃)** | **84.0** | **2200.0** | **26.190475** |
| **Sodium Chloride (NaCl)** | **58.0** | **6800.0** | **117.24138** |
| **Sodium Phosphate monobasic (NaH₂PO₄-H₂O)** | **138.0** | **140.0** | **1.0144928** |

| **Ribonucleosides** | | | |
|---|---|---|---|
| **Adenosine** | **267.0** | **10.0** | **0.037453182** |
| **Cytidine** | **243.0** | **10.0** | **0.041152265** |
| **Guanosine** | **283.0** | **10.0** | **0.03533569** |
| **Uridine** | **244.0** | **10.0** | **0.040983606** |

| **Deoxyribonucleosides** | | | |
|---|---|---|---|
| **2'Deoxyadenosine** | **251.0** | **10.0** | **0.03984064** |
| **2'Deoxycytidine HCl** | **264.0** | **11.0** | **0.041666668** |
| **2'Deoxyquanosine** | **267.0** | **10.0** | **0.037453182** |
| **Thymidine** | **242.0** | **10.0** | **0.041322313** |

| **Other Components** | | | |
|---|---|---|---|
| **D-Glucose (Dextrose)** | **180.0** | **1000.0** | **5.5555553** |
| **Lipoic Acid** | **206.0** | **0.2** | **9.708738E-4** |
| **Phenol Red** | **376.4** | **10.0** | **0.026567481** |
| **Sodium Pyruvate** | **110.0** | **110.0** | **1.0** |

GlutaMAX (registered trademark, GIBCO) added to "HTM-alpha" is a 200 mM solution ("GlutaMAX") in which L-alanyl-L-glutamine, as a dipeptide substitute for L-glutamine, is dissolved in physiological saline (0.85N NaCl). It is marketed as "GlutaMAX^{™} I (100 ×)". This was added into "HTM-alpha" so as to be diluted by 1/20 to become 10 mM (5 ×) when using.

With regard to the UW solution, HTM1 to HTM2 and HTM-alpha in the above, and the commonly used culture solution Earls BSS (EBSS -Earle's Balanced Salt Solution, ThermoFisher scientific); sodium ion concentration, potassium ion concentration, and molar ratios between these two are listed in Table 3. It should be noted that StemFit (registered trademark) AK02N and MEM-alpha also have the same ion concentration as Earls BSS. In view of experimental results described later, it is considered that the molar ratio of Na⁺/K⁺ should be around 1 or a little smaller.

**[Table 3] Molar ratio of Na⁺/K⁺ in commercially available medium and media of Examples**

| | **UW solution** | **Earls BSS (StemFit^{®}, and MEM-alpha)** | **HTM1, HTM2 and HTM-alpha** | **HTS-FRS** |
|---|---|---|---|---|
| **Na⁺ (mM)** | 25.0 | 116.4 | 55.5 | 100.0 |
| **K⁺ (mM)** | 125.0 | 5.4 | 85.1 | 42.5 |
| **Na/K** | 0.2 : 1 | 21.7 : 1 | 0.7 : 1 | 2.3 : 1 |

As mentioned above, proteins, peptides, and growth factors are contained in StemFit^{®} AK02N, but not in MEM-alpha. Therefore, proteins, peptides, and growth factors are contained in HTM1, but not in HTM-alpha.

### 3.3. Non-freezing refrigerated storage in single cell state

The human iPS cells obtained in "1." above are added into the refrigerated storage liquids HTM1 and HTM2 in "2." above to be suspended at 1E6 cells/mL (number of floating cells 1×10⁶ cells/mL) in a cell state. Then, the suspensions was placed into a plastic tube and allowed to stand at 4 °C. for storage. The survival rate was determined by counting the number of living cells after staining with trypan blue.

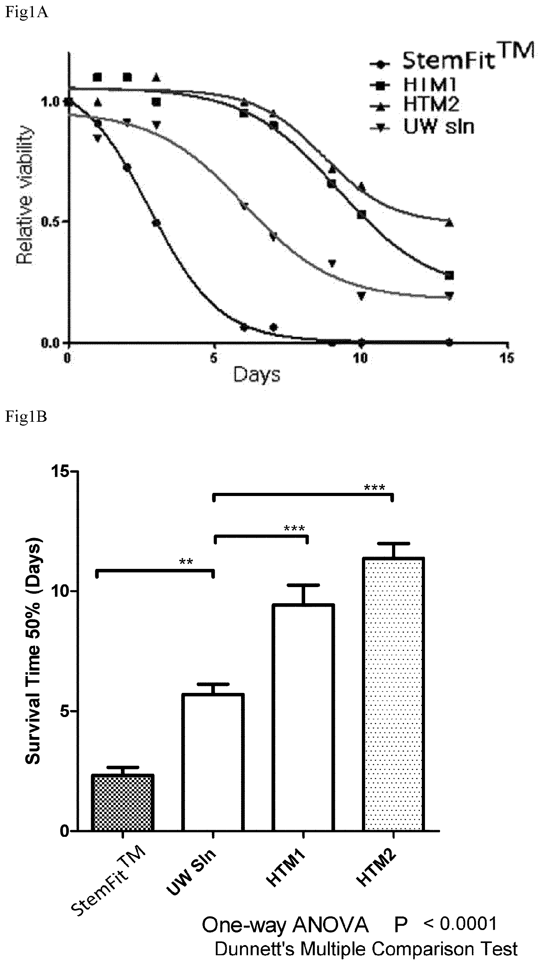

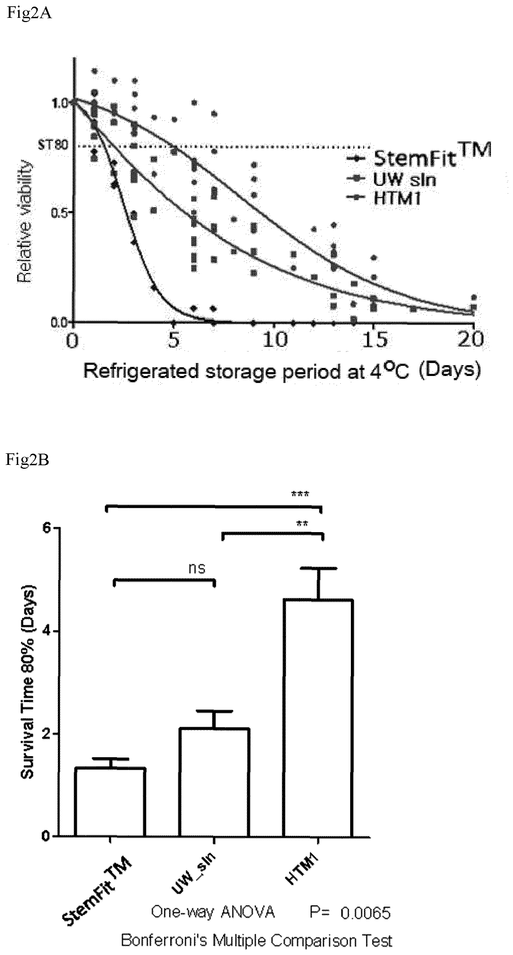

The results of survival rate measurement over time are shown in FIGS. 1A and 2A. From the graph of FIG. 1A, the 50% survival-rate maintenance days (ST50) was obtained and shown in FIG. 1B. Further, the 80% survival-rate maintenance days (ST80) was obtained from the graph of FIG. 2A and shown in FIG. 2B.

The statistical processing in the examples of the present application was performed as follows. The survival rate maintenance curves in FIGS. 1A and 2B were obtained by using "R ver 3.5.1" to fit the four-variable logistic curve. The 1way ANOVA and Bonferoni and Dunnetts tests were calculated using "Graphpad Prism 5.04".

As shown in FIGS. 1A and 2A, higher survival maintenance effects were obtained by HTM1 (UW solution + StemFit (registered trademark), 2/1 volume ratio) and HTM2 (UW solution + "ESC medium", 2/1 volume ratio), which are refrigerated storage liquids of the present embodiment, as compared with the UW solution. As shown in FIGS. 1A and 1B, by adopting the storage liquid of HTM2, 50% survival-rate maintenance days (ST50) was extended to about twice of that of the UW solution. With regard to HTM1 and HTM2 in comparison, the 50% survival-rate maintenance days (ST50) were almost same with each other and were significantly higher than that of the UW solution. As shown in FIGS. 2A and 2B, when 80% survival-rate maintenance days (ST80) were compared, 4.6 days was achieved by HTM1, which was remarkably high and 2.2 times of a value of 2.1 days achieved by the UW solution.

Since the StemFit (registered trademark) medium used for HTM1 is free of animal-derived components (Xenofree), subsequent experiments were examined using HTM1. When the "ESC medium" was used as it was for non-freezing refrigerated storage, the survival maintenance effect was significantly lower than that of the UW solution. While "ESC medium" was used in experiments below, results are shown only in the graph and explanation will be omitted.

### 4. Colony forming ability maintenance effect

The cell proliferation ability after non-freezing refrigerated storage was verified as follows. First, the human iPS cells obtained in the above" 1." were placed into the refrigerated storage liquid HTM1 of the above "2." and stored in the non-freezing refrigerated state for 6 days as in the above "3.". The cells were then suspended in StemFit^{®} AK02N medium containing 10 µM Y27362, into which iMatrix-511 was added by 0.1 µg/cm², and seeded at a concentration of 1.3E3/cm². On 5th day of culturing, the cells were stained with fixed alkaline phosphatase, and then the number of colonies formed to a certain size or larger was measured using a colony counter. The number of colonies when cultured for 5 days without being subjected to the refrigerated storage was similarly measured and used as a control. Then, the number of colonies obtained by culturing for 5 days after the refrigerated storage was divided by the number of colonies in this control to obtain colony forming ability (Relative colony efficiency).

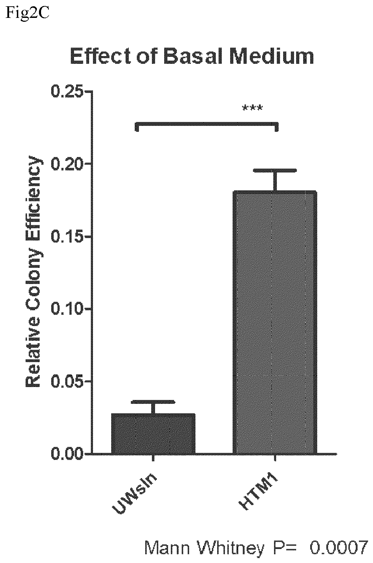

As shown in FIG. 2C, a value obtained by HTM1 was significantly higher than, and 6.7 times of, that obtained by the UW solution. From results hereto, it was found that the novel non-freezing cell refrigerated liquid HTM1 has a high viability maintenance effect and an effect of maintaining proliferative ability after recultivation, in the low temperature storage of human iPS cells. No colony formation was observed when StemFit^{®} AK02N was used.

### 5. Detection and measurement of apoptotic cells

The human iPS cells obtained in the above "1." are placed in the refrigerated storage liquid HTM1 of the above "2." and stored as in the above "3." for 3 days; and the cell death occurring immediately after this is classified by using Annexin V/EthD-III. That is, in order to distinguish apoptotic cells and necrotic cells from the others, firstly, the cells were stained according to the protocol by using the Apoptotic/Necrotic Cells Detection Kit (TAKARA). Then, Annexin V-FITC positive cells (apoptosis), ethidium homodimer III positive cells (necrosis), and Hoechst33342 (Dojin) signal (whole cells) were observed and measured under a fluorescence microscope.

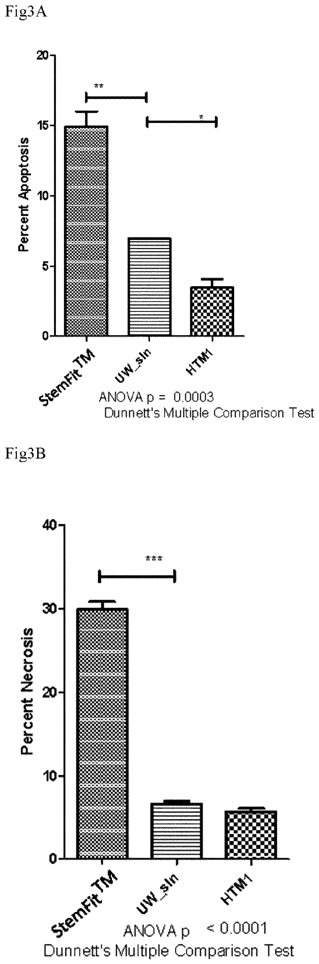

This results are shown in bar graphs of FIGS. 3A and 3B. As shown in FIG. 3A, it was known that when HTM1 was used, the induction of apoptosis was significantly suppressed as compared with the case where UW solution was used. It is considered that HTM1 has a higher proliferative ability (colony forming ability) than the UW solution due to such an effect of suppressing apoptosis immediately after the storage.

### 6. Intracellular ROS measurement

In the refrigerated storage, it is important to maintain viability during the refrigerated storage period, but it is more important to suppress cell death that occurs during recultivation after the storage. In order to elucidate the cause of cell death that occurs after the start of low-temperature storage and recultivation, intracellular reactive oxygen species (ROS) were measured by using Aminophenyl Fluorescein (APF). Specifically, the measurement was performed as follows. Firstly, Aminophenyl Fluorescein (APF) (five ridger) was added to the medium after the refrigerated storage for 3 days in the same manner as in "5." above so as to achieve final concentration of 5 µM. And, addition here was made throughout a period of 30 minutes in an incubator of 5% CO₂, 5% O₂ and 37°C. Subsequently, the culture medium was replaced, and in same time, 10 µM of Hoechst33342 (Dojin) was added to perform counter-staining, and then image of the counter-staining was observed with a fluorescence microscope and photographed. The fluorescence signal of each cell was quantified by using the image analysis software ImageJ.

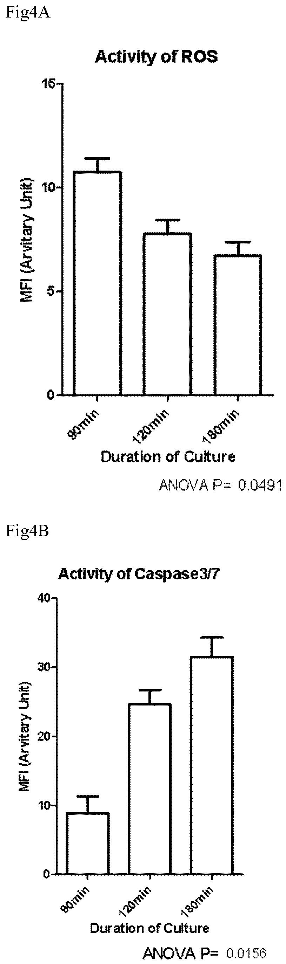

This result is shown in FIG. 4A. It was found that the intracellular ROS concentration tends to rise sharply within 90 minutes after the start of recultivation and then gradually decrease.

### 7. Measurement of intracellular caspase activity

In the same manner as in "5." above, the activity of intracellular caspase 3/7, which is an index of apoptosis, was measured for cells that had been stored for 3 days in the refrigerated state as in the above. First, Staining was performed according to the protocol using CellEvent ^{™} Caspase-3/7 Green Detection Reagent (Thermo Fisher). Then, the fluorescence signal of each cell was quantified using the image analysis software ImageJ.

This result is shown in FIG. 4B. It was found that the Caspase-3/7 activity, which is an indicator of apoptosis, gradually increased after 90 minutes. This result indicates that apoptosis is induced by a transient increase in intracellular reactive oxygen species (ROS) at the refrigerated storage and the start of recultivation.

### 8. Antioxidant or radical scavenger addition test

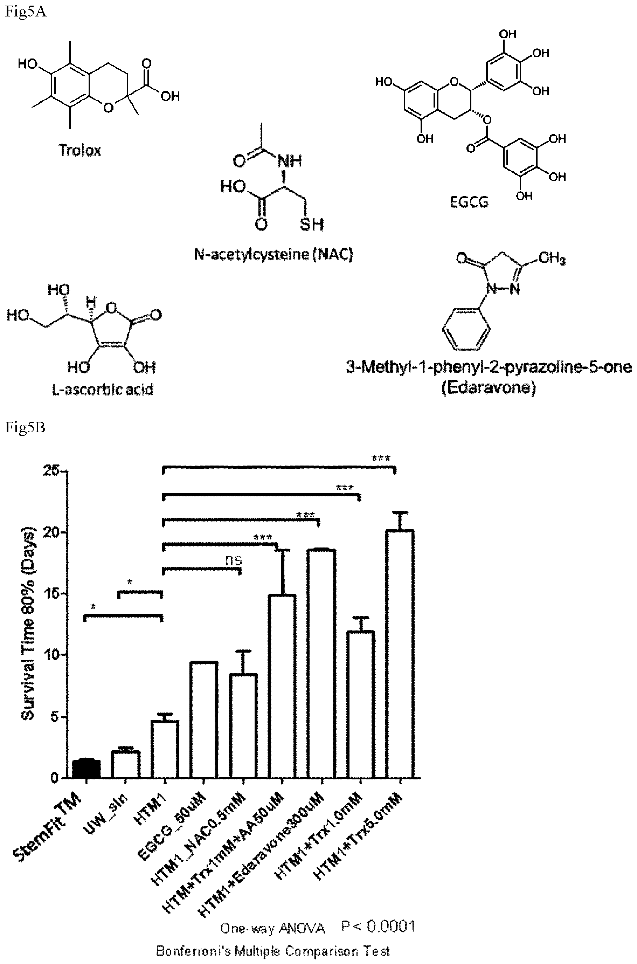

Antioxidants or radical scavengers were added to HTM1 at various concentrations and subjected to the non-freezing refrigerated storage test as described in "3." above. The added antioxidants or radical scavengers are shown in FIG. 5A and are: Trolox (Trx, Wako), EGCG (Teavigo^{®}, DSM), N-acetylcysteine (NAC, Tokyo Kasei), L-ascorbic acid (AA, WAKO) ), and edaravone (3-Methyl-1-phenyl-2-pyrazoline-5-one, Sigma).

FIG. 5B shows the results of comparing the 80% survival-rate maintenance days in the same manner as in FIGS. 2A to 2B. As shown in FIG. 5B, most effective was the addition of the water-soluble vitamin E-analog--trolox (5 mM), which extended the survival rate maintenance days to 9.5 times that of UW solution and 4.4 times that of HTM1 alone, and maintained a survival rate over 80% for about 20 days (ST80: 20.11 days). Next to trolox, Edaravone, which is known as a powerful radical inhibitor, has the second highest effect, and EGCG and N-acetylcysteine (NAC) have the third highest effect. L-Ascorbic Acid (AA), which was expected to work in concert with trolox, did not show much effect. Based on the above results, subsequent experiments were conducted focusing on the addition of trolox, which is highly versatile and can be used at a low price.

### 9. Addition concentration of trolox

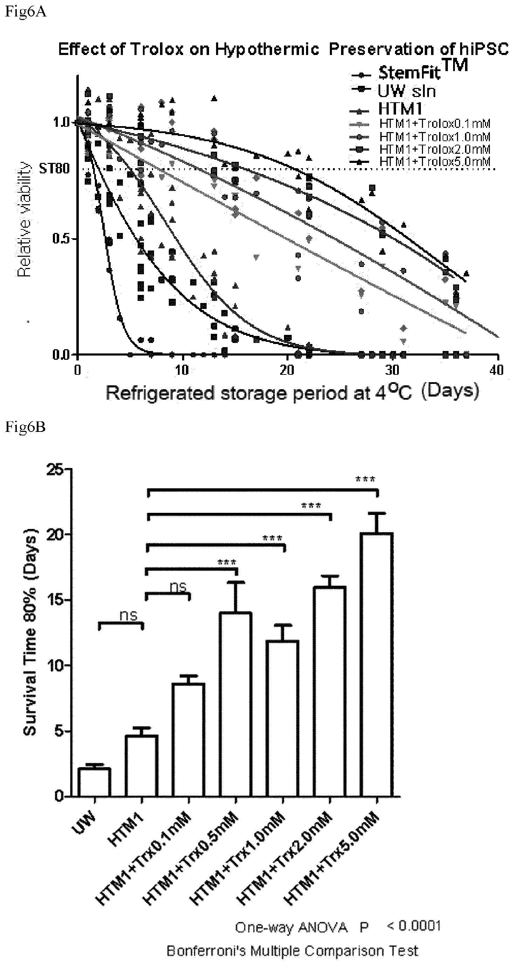

The effect of trolox, which had the highest viability-maintaining effect, was further investigated by varying the addition concentration from 0.1 mM to 0.5 mM. FIG. 6A shows the change over time in the survival rate as obtained in the same manner as in FIG. 2A, in a form superimposed on FIG. 2A. Further, FIG. 6B shows the results of determining the number of days for maintaining the 80% survival rate, similarly to FIGS. 2B and 5B. As shown in FIG. 6B, the 80% survival-rate maintenance days were found to increase approximately with increasing trolox concentrations. The 80% survival-rate maintenance days were significantly higher when 0.5 mM or more was added than when no trolox was added to HTM1.

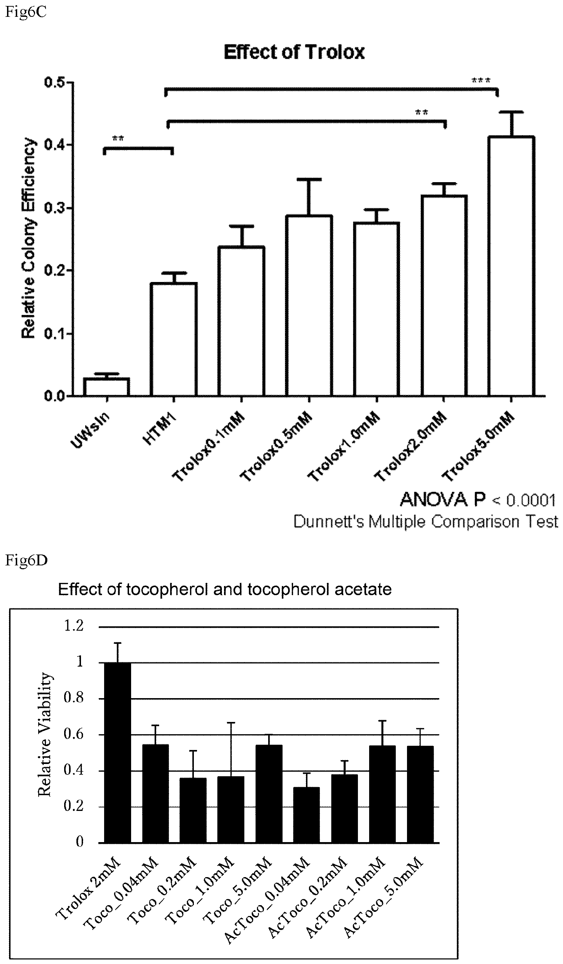

Next, in order to verify the proliferative ability after refrigerated storage and recultivation, cells having been stored in the refrigerated state for 6 days were seeded in the same manner as in "4." above, and the colony forming ability on the 5th day of culturing was compared. As shown in FIG. 6C, it was found that the colony forming ability evaluated in the same manner as in "4." above also increased depending on the concentration of trolox. The most effective colony forming ability was found in a group of the trolox 5.0 mM, and its colony forming ability was 2.3 times that of HTM1 alone, and was 41.2% of that of the control, which had not been subjected to the refrigerate storage. This obtained value was almost the same as that of the customary conventional slow-cooling cryoprerservation method placing the cell suspension in a freezer at - 80 °C. This indicates that by adding 5 mM of trolox to HTM1, the proliferation ability equal to or higher than that of cryopreservation can be maintained for up to 6 days.

### 9A. Comparison with the addition of Vitamin E and Vitamin E Derivatives

The HTM-alpha (UW solution + MEM-alpha, 2: 1) described in "2." above, which has not been added with trolox and GlutaMAX (registered trademark, GIBCO), is used in FIGS. 6A and 2A. The effect of the addition of tocopherol (vitamin E) and tocopherol acetate (vitamin E derivative) in the non-freezing refrigerated storage was investigated by the same procedure as in case of trolox addition in the above. Tocopherol and tocopherol acetate were added so as to become 0.04 mM to 5.0 mM in the same manner as the trolox addition, and refrigerated storage was performed. At this time, a sample to which trolox at 2.0 mM, which is a water-soluble vitamin E analog, was added was also tested at the same time. FIG. 6D summarizes the relative survival rates after 6 days of the storage and 1 day of recovery cultivation. From this result, it was found that both tocopherol (vitamin E) and tocopherol acetate are less effective in refrigerated storage than trolox.

### 10. Refrigerated storage in adhesive state (1)

Human iPS cells obtained in the same manner as in "1." above were used. However, at the final stage of culturing, the cells were seeded at a slightly lower concentration (1.0E3/cm²) than the maintenance culture of "1." above, and the day after this seeding, culture medium was replaced by StemFit^{®} AK02N medium containing no Y27362. Then, the medium was removed 4 days after the seeding.

Subsequently, the medium was replaced with HTM1 which is for the non-freezing refrigerated storage, and the medium with the cells was left to stand for 3 or 6 days under 4 °C. In other words, in a state the cells were aggregated and adhered to each other, the cells were stored in non-freezing refrigerated condition for 3 days or 6 days. Trolox was added to HTM1, which is the medium for refrigerated storage. In addition, adenine hydrochloride (Tokyo Kasei) was added to such media so as to become 0 mM, 1.4 mM and 4.1 mM, respectively. A UW solution (BELZER UW (registered trademark) COLD STORAGE LIQUID) was also used as the refrigerated storage medium of the comparative example.

The cells after the non-freezing refrigerated storage were lightly washed with culture medium after the refrigerated storage medium were removed. Then, StemFit^{®} AK02N medium was added and culturing was made for 4 days. At the end of this culturing, cells were enzymatically detached (by using 0.5X TrypLE^{®} Select), and dispersed so that number of viable cells are counted. Then, it was compared with the number of cells at the start of refrigerated storage. In addition, after taking a phase difference image at the end and start of the culturing, image processing was performed with ImageJ to extract and binarize the colony area, and the colony area was measured to evaluate the proliferative ability.

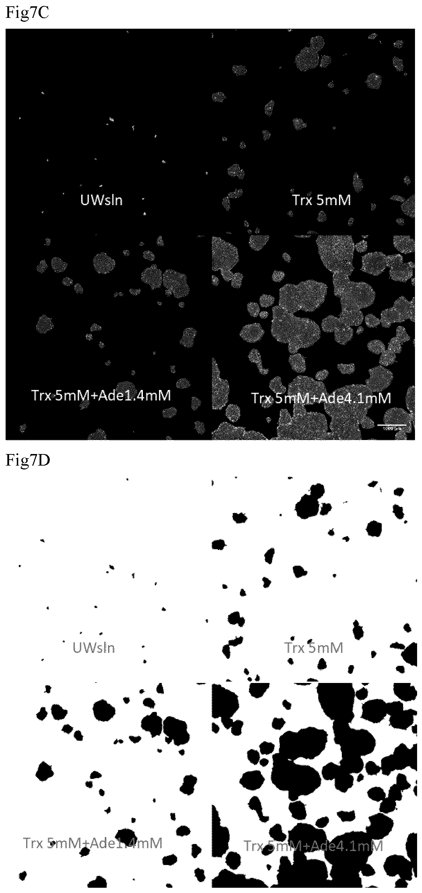

The above experiment was aimed at developing a refrigerated storage method in a state iPS cells were adhered, and proliferative ability (colony forming ability) subsequent to culturing for 4 days after the non-freezing refrigerated storage for 3 or 6 days. The results of the experiment are shown in FIGS. 7A-7F and 8. FIGS. 7A-7B show images of colonies obtained by the refrigerated storage for 4 days after 3 days of the refrigerated storage, and FIGS. 7C-7D show images of colonies obtained by recultivation for 4 days after he refrigerated storage for 6 days. Here, FIGS. 7A and 7C are phase differentiation images, and binarized images of these are shown in FIGS. 7B and 7D, respectively.

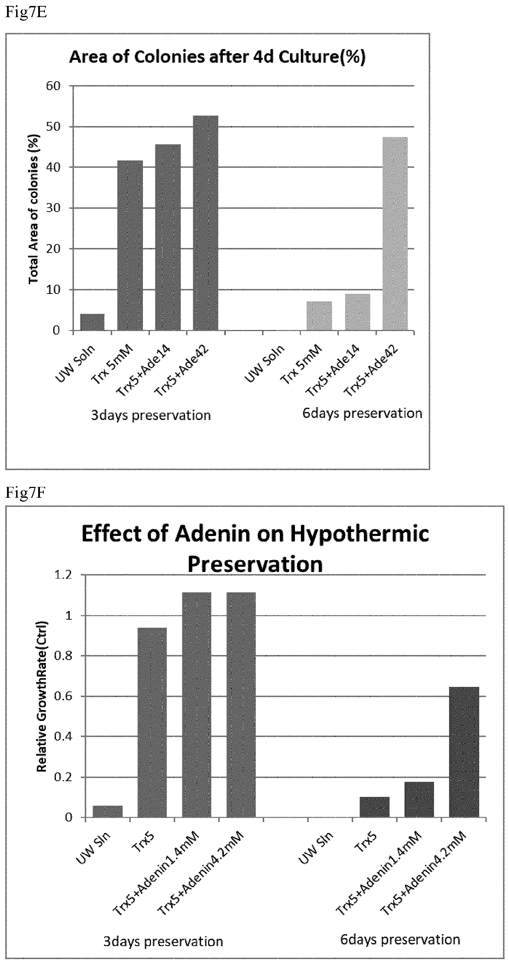

In addition, the bar graph in FIG. 7E shows the area ratios of colonies in media recultivated for 4 days after the refrigerated storage for 3 days and 6 days. Further, in the bar graphs of FIGS. 7F and 8, the rate of increase in the number of colonies when recultivated for 4 days after the refrigerated storage for 3 days and 6 days. In the graph, the rate for a control, which is for cell not subjected to the refrigerated storage, is taken as "1" or 100%. The bar graph of FIG. 8 also shows the experimental results, after the non-freezing refrigerated storage for 6 days, by using HTM1 as it is (without adding Trolox, etc.) and that using a refrigerated medium, which was obtained by adding trolox to HTM1 so as to become 8.2 mM.

As shown in the upper left part of each of FIGS. 7A to 7D, when stored as refrigerated in the UW solution, almost no proliferation was observed during recultivation after the non-freezing refrigerated storage for 3 or 6 days. When stored as refrigerated for 3 days by using a refrigerated storage medium obtained by adding Trolox 5 mM into HTM1, considerable growth was observed as shown in the upper right part of each of FIGS. 7A to 7B. And, as shown in the left part of FIG. 7F, the cell proliferation rate at the recultivation after the refrigerated storage for 3 days showed almost the same value as that in the control not subj ected to the refrigerated storage. On the other hand, when stored as refrigerated for 6 days by using the refrigerated storage medium, which is obtained by adding Trolox 5 mM into HTM1, some proliferation was observed as shown in the upper right part of each of FIGS. 7C to 7D. As shown in the right part of FIG. 7F, the cell proliferation rate when recultivated after the refrigerated storage for 6 days was only about 10% of the control not subjected to the refrigerated storage.

On the other hand, as shown in the lower left and lower right parts of FIGS. 7A to 7D, it was possible to increase the growth rate by further adding adenine to HTM1 together with Trolox 5 mM. In particular, as shown in FIG. 8, when the adhered cells were stored as refrigerated for 6 days, the proliferation rate at a time of recultivation was found to be significantly increased by adding 2.8 mM and 4.2 mM of Adenine in addition to Trolox 5 mM. It was found that the addition of adenine increased the growth rate with the increase in concentration up to 4.2 mM, but the addition of 8.2 mM decreased the growth rate.

### 11. Refrigerated storage in adhesive state (2)

By usiing the HTM-alpha (UW solution + MEM-alpha, 2: 1) described in the above "2.", the refrigerated storage in the adhesive state was evaluated by the same operation as in the above "10.". However, the evaluation was made subsequent to recultivation after the refrigerated storage for 7 days instead of 6 days. Here, as described above, 5 mM Trolox and 5-fold concentration (x5; 10 mM) GlutaMAX^{®} (registered trademark, GIBCO) were added to HTM-alpha. In addition, HTM1 and HTS FRS were compared by using them at the same time under the same conditions. As with HTM-alpha, 5 mM Trolox and 5-fold concentration (x5; 10 mM) GlutaMAX^{®} (registered trademark, GIBCO) were added to HTM1.

This result is shown in the graph of FIG.9. In the bar graph of FIG. 9, similarly to FIGS. 7E and 8, the ratios were taken by that the non-preserved control cultured for 4 days without refrigerated storage was set to 1. As shown in the graph of FIG. 9, when HTM-alpha (middle one) was used as the refrigerated storage medium, the growth rate was significantly higher than that when HTM1 was used. In addition, it showed a much higher growth rate than the case of using HTS FRS, which is a commercially available refrigerated storage liquid.

From this result, it is considered that fibroblast growth factor (FGF) contained in the C solution of StemFit (registered trademark) AK02N is unnecessary or does not contribute much. In addition, among the components contained in StemFit (registered trademark) AK02N, those not contained in HTM-alpha were considered to be not essential. In particular, since HTM-alpha is a protein-free medium, it was considered that protein is not essential or should be absent.

### 12. Refrigerated storage of mouse embryos

As described above, a blastocyst stage embryo 3.5 days after in vitro fertilization was immediately used as it was, and a refrigerated storage experiment was conducted. Specifically, for this blastocyst stage embryo, the medium was replaced with the HTM-alpha, HTM1, and HTS FRS as used in the above "11." and the UW solution, in respective experiments. Thus obtained suspension was then placed in a plastic tube and was allowed to be kept at 4 °C for 3 days (72 hours). Both of HTM-alpha and HTM1 here are obtained by adding 5 mM Trolox and 5-fold concentration (x5; 10 mM) GlutaMAX (registered trademark) in the same manner as in "11." above.

FIG. 10 shows the rate of embryos that escaped from the zona pellucida during the culture period of 2 days, which is a further culturing period. FIG. 10 also shows the results of refrigerated storage and culturing for the unpreserved control (F_Control) that was not stored as refrigerated.

As shown in FIG. 10, both HTM1 and HTM-alpha showed the same escape rate as the unpreserved control (F_Control), but both UW solution and HTS FRS showed a significantly lower escape rate.

Next, the effect of adding adenine hydrochloride (Tokyo Kasei) was investigated in the same manner as in FIGS. 7E to 7F. That is, under the same conditions as in the experiment of FIG. 10, the rate of embryos that escaped the zona pellucida when Adenine hydrochloride (Tokyo Kasei) was added at each concentration was determined. FIG. 11 shows the results when unpreserved control (F_Control) and adenine hydrochloride were added so as to become 1.4 mM (1400 µM), 140 µM, 14 µM, and 0 µM, respectively, in order from the left. As is known from FIG. 11, by way of the non-freezing low temperature storage for 5 days and 7 days, a relatively high zona pellucida escape rate was exhibited when the Adenine concentration was 14 µM to 140 µM (0.014 to 0.14 mM).

### 13. Effect of addition of polyvinyl alcohol (PVA)

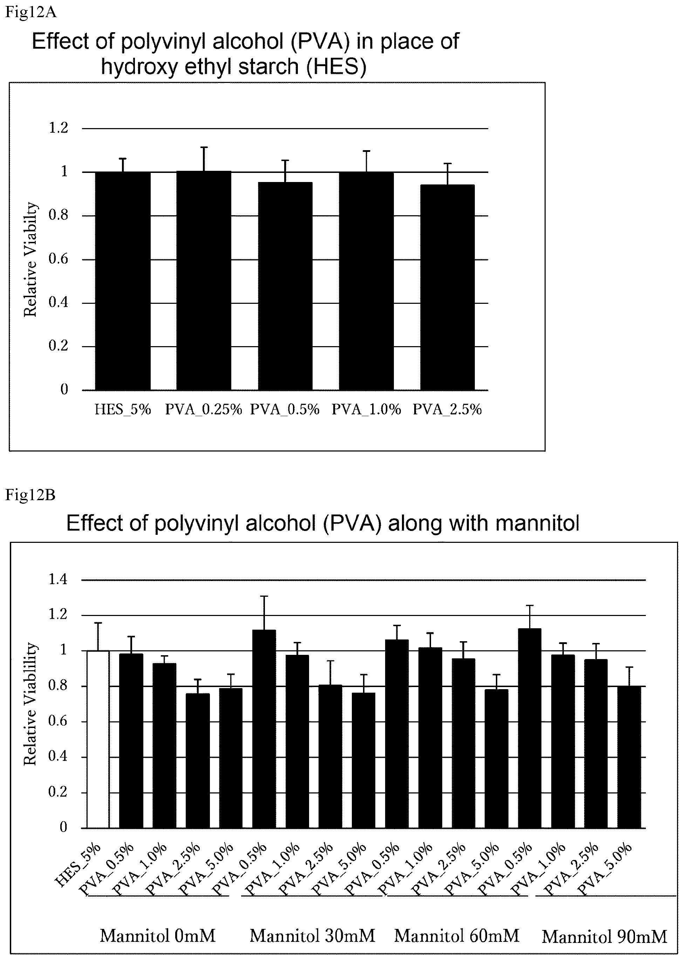

As shown in Table 1 above, since the UW solution contains 5.0% hydroxy ehyl starch (HES), HTM-alpha (UW solution + MEM-alpha, 2/1) described in "2." contains 3.3% hydroxyethyl starch (HES). Here, the effect of adding polyvinyl alcohol (PVA) instead of hydroxy ethyl starch (HES) was investigated. Here, the composition of the UW solution shown in Table 1 above, excluding hydroxyethyl starch (HES), was adopted. Thus, the other components are same with those in the HTM-alpha (UW solution + MEM-alpha, 2: 1) while polyvinyl alcohol (PVA) in the range of 0.25% to 2.5% (weight) were added in the storage liquids used here. The cell viability was evaluated in the same manner as in the case of FIG. 6D above. That is, the protective effect of polyvinyl alcohol (PVA) was examined by the same procedure as in FIGS. 6A and 2A. At this time, a sample having a hydroxyethyl starch (HES) concentration of 5% (weight) was also tested at the same time. FIG. 12A summarizes the relative survival rates after 6 days of the refrigerated storage and 1 day of recovery culturing. From this results, it was found that polyvinyl alcohol (PVA) shows the same protective effect as 5% hydroxyethyl starch (HES) in any concentration range. It should be noted that polyvinyl alcohol (PVA) is cheaper than hydroxyethyl starch (HES), and is considered to be safer for living tissues.

### 14. Effect of addition of mannitol

Next, in the same manner as in "13." above, mannitol was added together with polyvinyl alcohol (PVA), and the synergistic effect was verified. That is, polyvinyl alcohol (PVA) was added in the range of 0.5% to 5% to HTM-alpha (UW solution + MEM-alpha, 2: 1) without hydroxyethyl starch (HES), and cell membrane impermeable. Mannitol, which is considered to have a sexual osmoregulatory effect, was added with 0, 30, 60, and 90 mM. In addition, the protective effect was examined by the same procedure as in the cases of FIGS. 6A and 2A. FIG. 12B shows in a summarized manner, the relative survival rates after 6 days of the refrigerated storage and 1 day of recovery culturing, as in the case of FIG. 12A. According to the results of FIG. 12B, when mannitol was added in the range of 30 mM to 90 mM together with 0.5% polyvinyl alcohol (PVA), it was found that the viability of the cells after refrigerated storage were higher than those obtained by the storage liquid containing hydroxyethyl starch (HES) at a concentration of 5%.

### 15. Summary of experimental results

When the cell cryopreservation medium HTM1 of the present embodiment in which the UW solution and the commercially available clinical research medium StemFit^{®} AK02N were mixed at a ratio of 2/1 was used, as shown in FIGS. 1B and 2B, it was able to increase the survival-rate maintenance period in the non-freezing refrigerated storage of human iPS stem cells at a low temperature of 4 °C, to about two times of that obtainable by the UW solution, which is widely used as a cryopreservation solution. Moreover, as shown in FIG. 6C, the cell proliferation ability after the refrigerated storage for 6 days and recultivation was enhanced to about 7 times that of the UW solution.

In addition, it is considered that the addition of an antioxidant/radical scavenger to HTM1 made it possible to suppress the rapid increase in ROS concentration that occurs at the start of recultivation after the refrigerated storage. With the addition of Trolox 5 mM, as shown in 6A and 6B, the maintenance period of 80% survival rate could be 20 days or more, which was about 4 times that of HTM1 without Trolox addition. In addition, as shown in FIG. 6C, it was confirmed that the colony formation rate after the refrigerated storage and recultivation was increased to about twice that of the additive-free groups.

On the other hand, when cryopreserved in the adhered state, it was clarified that the addition of Adenine in addition to the addition of trolox increased the subsequent growth rate, as shown in FIGS. 7A-7F and 8.

On the other hand, when the cell cryopreservation medium "HTM-alpha" (UW solution + MEM-alpha, 2/1) of a particularly preferable embodiment obtained by mixing UW solution and MEM alpha was used; as shown in FIG. 9, it was considered that the effect of maintaining the cell proliferation ability was greater than that of HTM1 (UW solution + StemFit (registered trademark) AK02, 2/1).

Although the storage period of iPS cells is limited as compared to frozen storage, iPS cell storage can be stored in a state of culturing and adhered state; and differentiation-induced organoids that cannot be frozen can be stored and transported. Or, it is a technology with a very high range of applications, such as on-demand cell supply in drug discovery research. Therefore, this technology, which enables the preservation of human iPS cells, is extremely important.

In view of that the cell refrigerated storage medium "HTM-alpha" of the embodiment of the present application was effective in storing mouse embryos in the non-freezing refrigerated state, the cell refrigerated storage medium of the embodiment would also be or may effective for strong the embryonic or non-embryonic stem cells of humans and various animals and tissues in the non-freezing refrigerated state.

## Claims

1. A refrigerated storage liquid for storing human or animal stem cells or embryos in a non-frozen state, comprising:
potassium ion species in a range of 30 to 80 mmoL/L;
sodium ion species in a range of 30 to 80 mmoL/L, wherein ion-based molar ratio of sodium ion species to potassium ion species (ratio of Na⁺ to K⁺) is in a range of 0.5 to 1.3;
Trolox or its analog at a concentration of 0.5 to 8 mM;
adenine or a salt or derivative thereof at a concentration of 0.1 to 4.2 mM;
all of or all but one, two or three kinds of essential amino acids, total content of which is 50 to 200 mg; and
non-essential amino acids, total content of which is 50 to 200 mg/L.

2. A refrigerated storage liquid for storing human or animal stem cells or embryos in a non-frozen state, comprising:
(i) lactobionic acid or its salt in terms of lactone in a range of 30 to 100 mmoL/L,
(ii) raffinose hydrate in a range of 10 to 30 mmoL/L,
(iii) alloprinol in a range of 0.3 to 1 mmoL/L,
(iv) glutathione (total glutathione) in a range of 1 to 3mmoL/L,
(v) adenosine in a range of 2 to 10mmoL/L,
(vi) lipoic acid in a range of 0.1 to 0.5µmoL/L,
(vii) sodium pyruvate in a range of 0.1 to 0.7mmoL/L,
(viii) glucose in a range of 1 to 5mmoL/L,
(ix) ascorbic acid in a range of 0.03 to 0.3 mmoL/L and vitamin E or its water-soluble analog/derivative in a range of 0.5 to 8 mM,
(x) adenine or its salt or derivative in a range of 0.1 to 4.2 mM,
(xi) other vitamins or its water-soluble analog/derivatives, including folic acid, nicotine amide, riboflavin, B 12, choline, inositol, pantothenic acid, pyridoxal phosphate and thiamine,
(xii) essential amino acids 50 to 200 mg/L in total,
(xiii) non-essential amino acids, in a range of 100 to 500 mg/L in total,
(xiv) potassium ion species in a range of 30 to 80 mmoL/L, and
(xv) sodium ion species in a range of 20 to 90 mmoL/L.

3. The refrigerated storage liquid according to claim 1 or 2, further comprising:
20 to 50 g/L of hydroxy ethyl starch,
5 to 50 g/L of polyvinyl alcohol, and/or
30 to 90 mmoL/L of mannitol.

4. The refrigerated storage liquid according to any one of claims 1 to 3, further comprising L-alanyl-L-glutamine or another dipeptide substitute, or a salt thereof, at a concentration of 2 to 5 mM.

5. A refrigerated storage method comprising: suspending or immersing human or animal iPS cells or other artificial stem cells, other stem cells, organoids obtained from them, or embryos in the refrigerated storage liquid according to any one of claims 1 to 4; and storing the cells in such suspended or immersed state, at 2 to 8 °C for 1 to 10 days.
